# EUROPÄISCHE PATENTSCHRIFT

(11) **EP 2 091 447 B1**
(45) Veröffentlichungstag und Bekanntmachung des Hinweises auf die Patenterteilung: **06.04.2011**
(21) Anmeldenummer: 07822644.6
(22) Anmeldetag: 15.11.2007
(51) Int. Cl.: A61B 17/68, F16B 13/12

(54) **FIXATIONSELEMENT FÜR KNOCHENFRAGMENT**
FIXING ELEMENT FOR A BONE FRAGMENT
ÉLÉMENT DE FIXATION POUR FRAGMENT OSSEUX

(30) Priorität: 15.11.2006 DE 102006054533
(43) Veröffentlichungstag der Anmeldung: 26.08.2009
(73) Patentinhaber: Resoimplant GmbH, 80807 München (DE)
(72) Erfinder: LOB, Günter, 81377 München (DE)
(74) Vertreter: von Oppen, Joachim F.M.
(86) Internationale Anmeldenummer: PCT/EP2007/062410
(87) Internationale Veröffentlichungsnummer: WO 2008/059027

(56) Entgegenhaltungen:
- EP-A- 1 195 142
- DE-A1- 4 444 510
- US-A- 5 980 522

## Beschreibung

Die Erfindung betrifft ein implantierbares Befestigungselement, insbesondere einen Dübel, für die Fixation von beispielsweise Knochenfragmenten, also ein Befestigungselement für den chirurgischen Einsatz.

Das Befestigungselement ist in Längsrichtung in wenigstens zwei äußere Teilelemente geteilt und kann ein Spreizelement aufweisen. Derartige Befestigungselemente sind so gestaltet, dass sie mittels des Spreizelementes oder eines entsprechenden Werkzeugs von einem kompakten Zustand in einen (radial bzw. lateral) expandierten Zustand zu überführen sind.

Derartige Befestigungselemente, wie sie beispielsweise aus der WO96/16607 oder der US 5, 980, 522 bekannt sind, können nach Art eines Dübels zur Fixation von Bändern an einem Knochen oder zu Fixation von Knochenfragmenten verwendet werden. Dazu wird das Befestigungselement in eine Bohrung im Knochen eingesetzt und anschließend mit Hilfe des Spreizelementes gespreizt. Aufgrund der Spreizung wird das Befestigungselement an den beiden Knochenfragmenten fixiert und fixiert auf diese Weise auch die beiden Knochenfragmente relativ zueinander.

Um die beiden äußeren Teilelemente mittels eines geeigneten Instruments an ihrem jeweiligen proximalen Ende festhalten und an den gewünschten Ort führen zu können, sind verschiedene Koppelstrukturen bekannt, die es erlauben, das Befestigungselement mit dem Instrument zu koppeln. Die mit Hilfe der jeweiligen Koppelstruktur herzustellende Verbindung zwischen dem Befestigungselement und dem Instrument zu Einführen muss einerseits zuverlässig sein, um das Befestigungselement sicher an seinen Implantationsort führen zu können, sie muss anschließend aber auch zuverlässig trennend sein, um das Befestigungselement zuverlässig loslassen zu können.

Der Erfindung liegt die Aufgabe zugrunde, ein Befestigungselement der aus WO 96/16607 oder US 5,980,522 bekannten Art zu schaffen, das eine zuverlässige Koppelung an ein Instrument zu Einsetzen des Befestigungselementes erlaubt.

Erfindungsgemäß wird diese Aufgabe durch ein Befestigungselement der eingangs genannten Art gelöst, das an seinem proximalen Ende zur Koppelung mit einem Instrument zum Halten und Einführen des Befestigungselementes schwalbenschwanzartig ausgebildet ist und zwei auf gegenüberliegenden Seiten des Befestigungselementes sich quer zu einer Trennfläche zwischen den Teilelementen erstreckende Kerben aufweist, die sich jeweils über beide Teilelemente hinweg erstrecken und die derart geformt sind, dass die Tiefe der jeweiligen Kerbe zum proximalen Ende des Befestigungselementes hin abnimmt und so die Breite des Befestigungselementes aus einer Blickrichtung entlang der beiden Kerben zum proximalen Ende des Befestigungselementes hin zunimmt, wobei die Breite des Befestigungselementes an dessen proximalem Ende geringer ist, als in einem Längsabschnitt, der sich in distaler Richtung an die Kerben anschließt.

Die Kerben sind vorzugsweise jeweils von zwei Grenzflächen begrenzt, nämlich von einer distalen Grenzfläche, die senkrecht zur Längsachse des Befestigungselementes verläuft und von einer proximalen Grenzfläche, die in einem spitzen Winkel auf die Längsachse des Befestigungselementes zuläuft. Die Kerben habe also eine Art dreieckigen Querschnitt, der jeweils von zwei Außenflächen des Befestigungselementes gebildet wird, die schräg aufeinander zu laufen. Die oder US 5, 980, 522 distale Begrenzungsfläche verläuft vorzugsweise senkrecht zur Längsachse des Befestigungselementes, während die proximale Begrenzungsfläche vorzugsweise in einem Winkel von 60° +/- 5° zur Längsachse des Befestigungselementes verläuft.

Das Befestigungselement hat darüber hinaus in seinem kompakten Zustand über den größten Teil seiner Länge vorzugsweise eine zylindrische Grundform und lässt sich daher optimal in Rundbohrungen in ein Knochen einsetzen.

Vorzugsweise ist die Breite des Befestigungselementes an seinem proximalen Ende aufgrund der beiden Kerben um ein Maß geringer, als der Durchmesser der zylindrischen Grundform, das etwas mehr als der Tiefe einer Kerbe in radialer Richtung entspricht. Mit anderen Worten: jede Kerbe ist in Bezug auf den distal der kerbe gelegenen Längsabschnitt des Befestigungselementes knapp doppelt so tief, wie in bezug auf das proximale Ende des Befestigungselementes.

Bevorzugt ist ein Befestigungselement, welches in seinem kompakten Zustand eine zylindrische Grundform mit einem Durchmesser zwischen 3 und 7mm und eine Länge von über 60mm besitzt. In Längsrichtung des Befestigungselementes wechseln sich Längsabschnitte mit rundem Querschnitt und solche mit sternförmigem Querschnitt ab. Der sternförmige Querschnitt der entsprechenden Längsabschnitte wird durch prismenförmige Längsrippen auf der Außenseite dieser Längsabschnitte gebildet. Der Durchmesser der Längsabschnitte mit rundem Querschnitt ist geringer, als der Durchmesser der Längsabschnitte mit sternförmigem Querschnitt.

Durch Einführen des Spreizelementes, das in Bezug auf den von den Teilelementen eingeschlossenen Freiraum ein Übermaß besitzt, werden die beiden Teilelemente um ein durch das Übermaß des Spreizelementes exakt vorgegebenes Maß parallel auseinander getrieben.

Ein derartiges Befestigungselement lässt sich in seinem kompakten Zustand vorteilhaft in eine kreisrunde Bohrung einführen, die vom Knochenäußeren durch die Bruchfläche hindurch bis in einen weiteren Knochenteil hineinragt. Wird das erfindungsgemäße Befestigungselement von außen in eine derartige Bohrung eingeführt, kann es anschließend mit Hilfe des Spreizelementes expandiert werden und fixiert auf diese Weise die beiden Knochenteile über die Bruchfläche hinweg zueinander. Dabei ist es vorgesehen, dass das Befestigungselement eine Überlänge besitzt und somit proximal aus der Bohrung hinausragt. Der überstehende Teil des Befestigungselementes kann dann abgeschnitten werden.

Vorteilhafterweise besteht das Befestigungselement aus einem thermoplastischen Material, so dass das Befestigungselement mit Hilfe eines heißen Drahtes abgeschnitten werden kann. Dabei kommt es im günstigsten Fall zusätzlich zu einer Verschweißung zwischen den äußeren Teilelementen des Befestigungselementes und dem Spreizelement.

Ebenso ist es vorteilhaft, wenn das Material, aus dem die Elemente des Befestigungselementes bestehen, bioresorbierbar ist, so dass das Befestigungselement nach dem Heilen des Bruches vom Körper allmählich abgebaut wird. Ein geeigneter, biokompatibler Werkstoff ist beispielsweise ein Polylactid.

Das Befestigungselement kann darüber hinaus günstiger Weise Osteosynthese stimulierende Substanzen wie beispielsweise knochenmorphogene Proteine (bone morphogenic proteins) enthalten, die das Knochenwachstum weiter fördern. Idealerweise ist der Werkstoff des Befestigungselementes derart optimiert, dass die Abgabe solcher knochenwachstumsfördernder Substanzen in einer für den gewünschten Heilungsprozess geeigneten Dosis erfolgt.

In Bezug auf die äußere Geometrie des Befestigungselementes ist es günstig, wenn die Längsabschnitte mit rundem Querschnitt eine Länge zwischen 0,5 und 1,5mm besitzen, während die Längsabschnitte mit sternförmigem Querschnitt vorzugsweise eine Länge zwischen 1 und 3mm besitzen. Diese Geometrie erlaubt ein wirkungsvolles Verkrallen des expandierten Befestigungselementes im jeweiligen Knochen.

In Bezug auf die innere Geometrie des Befestigungselementes ist es günstig, wenn sowohl der Freiraum als auch das Spreizelement einen im Wesentlichen rechteckigen Querschnitt besitzen. Dabei ist der im Wesentlichen rechteckige Querschnitt des Spreizelementes an den Ecken vorzugsweise abgerundet. In Längsrichtung weist das Spreizelement distal einen sich zum distalen Ende hin verjüngenden Längsabschnitt auf, der es erlaubt, das Spreizelement mit diesem Längsabschnitt voran in den Freiraum zwischen den beiden äußeren Teilelementen des Befestigungselementes einzuführen, wenn dieses sich in seinem kompakten Zustand befindet.

Weiterhin ist es vorteilhaft, wenn die Trennfläche zwischen den beiden äußeren Teilelementen derart verspringt, dass die beiden Teilelemente auch im expandierten Zustand des Befestigungselementes wenigstens nicht in Längsrichtung zueinander verschieblich sind und möglichst auch nicht in lateraler Richtung. Dazu weist eines der beiden Teilelemente vorzugsweise über eine Haupttrennfläche hinausragende Vorsprünge auf, die in entsprechende Ausnehmungen in dem jeweils anderem Teilelement eingreifen. Derartige Vorsprünge sind vorzugsweise jedem dritten Längsabschnitt mit sternförmigem Querschnitt zugeordnet.

Die Erfindung soll nun anhand eines Ausführungsbeispiels mit Bezug auf die Figuren näher erläutert werden. Von den Figuren zeigen:
- Fig. 1:: eine Explosionsansicht des Befestigungselementes mit seinen beiden äußeren Teilelementen und dem Spreizelement;
- Fig. 2:: eine Ansicht des Befestigungselementes in seinem kompakten Zu- stand;
- Fig. 3:: verschiedene Detailansichten des Befestigungselementes aus Figur 1 und 2;
- Fig. 4:: eine Ansicht des Befestigungselementes in seinem expandierten Zustand;
- Fig. 5:: ein alternatives Befestigungselement in seinem kompakten Zustand
- Fig. 6:: verschiedene Detailansichten des alternativen Befestigungselemen- tes aus Figur 5; und
- Fig. 7:: eine Ansicht des alternativen Befestigungselementes in seinem ex- pandierten Zustand.

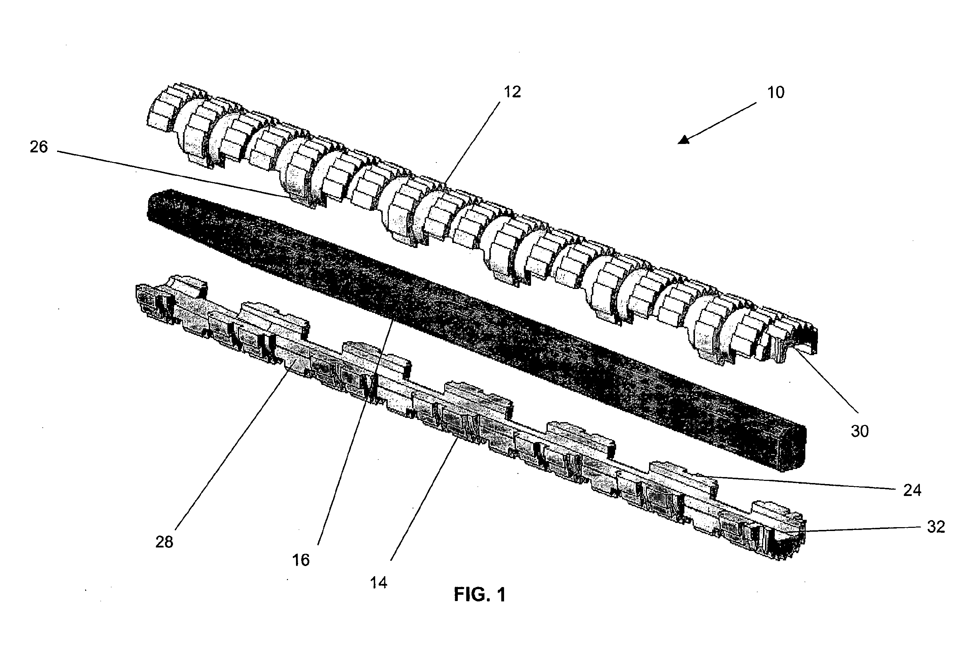

Fig. 1 zeigt eine bevorzugte Ausführungsvariante eines Befestigungselementes 10. Dieses Befestigungselement 10 besitzt zwei äußere Teilelemente 12 und 14, die ein Spreizelement 16 einschließen.

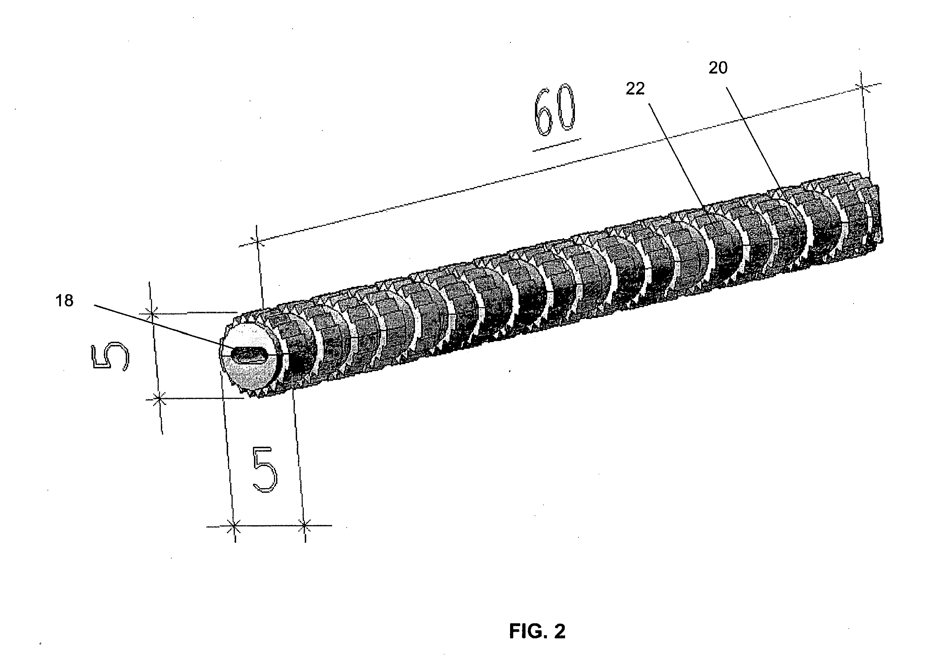

Fig. 2 zeigt das Befestigungselement 10 in seinem kompakten Zustand, in dem die beiden äußeren Teilelemente 12 und 14 unmittelbar aufeinander aufliegen. Zu erkennen ist, dass die beiden Teilelemente einen Freiraum 18 einschließen, in den das Spreizelement 16 eingeführt werden kann.

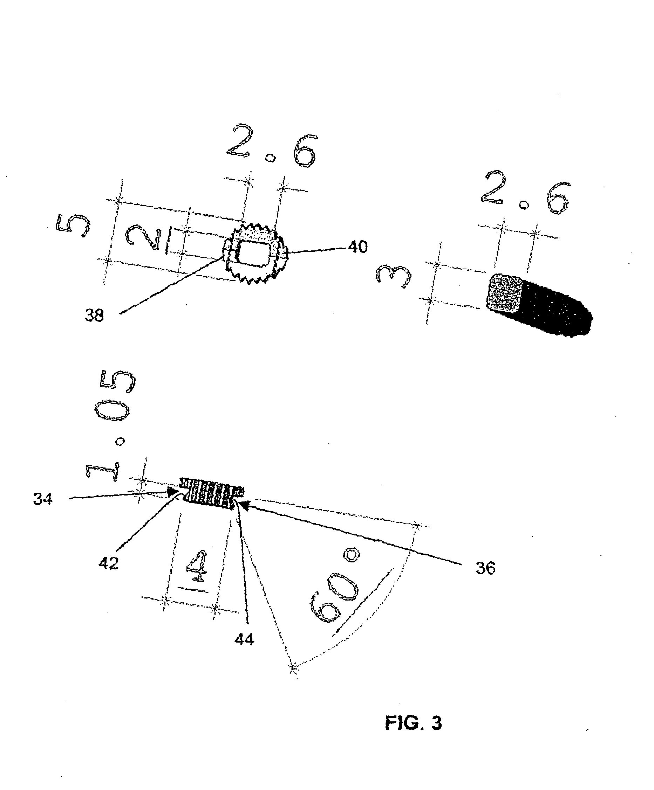

Die obere linke Abbildung der Fig. 3 ist ein Blick auf das proximale Ende des Befestigungselementes 10 in seinem komprimierten Zustand, wie es auch in Fig. 2 abgebildet ist. In Fig. 3 unten links abgebildet ist eine Draufsicht auf einen kurzen Längsabschnitt des Befestigungselementes 10 an dessen proximalen Ende. In dieser Draufsicht sind zwei Kerben 34 und 36 gut zu erkennen, in die ein Instrument zum Einführen des Befestigungselementes 10 angreifen kann, so dass ein solches Instrument über die Kerben 34 und 36 mit dem Befestigungselement 10 zu koppeln ist. Beide Kerben 34 und 36 werden jeweils von einer distalen Begrenzungsfläche 38 beziehungsweise 40 und einer proximalen Begrenzungsfläche 42 beziehungsweise 44 begrenzt. Die distalen Begrenzungsflächen 38 und 40 liegen dabei in einer Ebene, die senkrecht zur Längsachse des Befestigungselementes 10 verläuft. Die beiden proximalen Begrenzungsflächen 42 und 44 laufen jeweils in einem Winkel von 60° auf die Längsachse des Befestigungselementes 10 zu. Die Breite des Befestigungselementes in der in Fig. 3 unten links abgebildeten Blickrichtung entlang der Kerben 34 und 36 ist etwa 1 mm geringer, als die maximale Breite des Befestigungselementes 10 in einem Längsabschnitt der sich distal an die beiden Kerben 34 und 36 anschließt. Die minimale Breite des Befestigungselementes 10 am tiefsten Punkt der beiden Kerben 34 und 36 ist noch einmal etwa 1 mm geringer, als die Breite des Befestigungselementes 10 an seinem distalen Ende. Die in Millimeter angegebenen Maße einer bevorzugten Ausführungsform sind in den Figuren angegeben. Dies gilt auch für die Abbildung in Fig. 3 oben rechts, die eine perspektivische Darstellung des Spreizelementes 16 ist und eine Bemaßung von dessen Querschnitt aufweist. Den Abbildungen in Fig. 3 oben links und oben rechts ist klar zu entnehmen, dass das Spreizelement 16 in Richtung quer zu einer Trennfläche zwischen den beiden Teilelementen 12 und 14 ein Querschnittsmaß besitzt, das etwa 1 mm größer ist, als das Querschnittsmaß des von den beiden Teilelementen 12 und 14 eingeschlossenen Freiraums 18 in der entsprechenden Richtung.

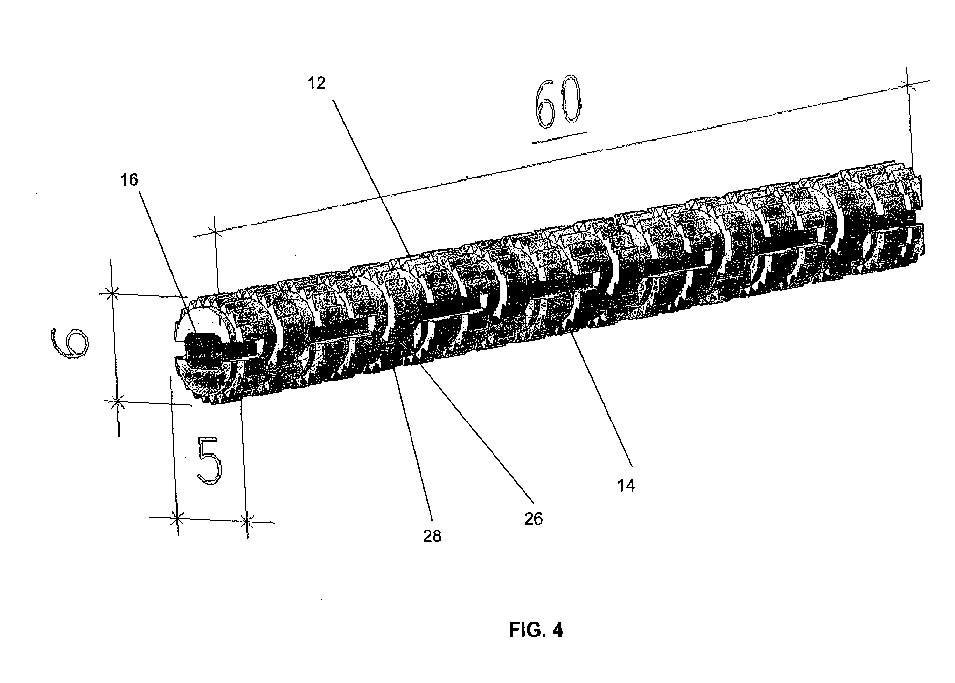

Fig. 4 zeigt das Befestigungselement 10 in seinem expandierten Zustand, bei dem das Spreizelement 16 in den Freiraum 18 eingeschoben ist.

Die beiden äußeren Teilelemente 12 und 14 sind so gestaltet, dass sie in dem in Fig. 2 dargestellten, kompakten Zustand des Befestigungselementes 10 diesem eine zylindrische Grundform verleihen, bei der sich Längsabschnitte 20 mit kreisrundem Querschnitt und Längsabschnitte 22 mit sternförmigem Querschnitt abwechseln. Das gesamte Befestigungselement 10 hat im Ausführungsbeispiel eine Länge von 60mm und einen maximalen Durchmesser im Bereich der Längsabschnitte 22 mit sternförmigem Querschnitt von 5mm. Der Durchmesser der Längsabschnitte 20 mit kreisrundem Querschnitt ist erkennbar etwas geringer als der Durchmesser der Längsabschnitte mit sternförmigem Querschnitt. Wie Fig. 2 ebenfalls zu entnehmen ist, rührt der sternförmige Querschnitt der Längsabschnitte 22 von prismenförmigen Längsrippen auf der Außenseite dieser Längsabschnitte her.

Die beiden äußeren Teilelemente 12 und 14 sind entlang einer Trennfläche 24 voneinander getrennt, die zu großen Teilen genau in der Mitte des Befestigungselementes 10 verläuft und eben ist. Auf Höhe eines jeden dritten Abschnitts 22 mit sternförmigem Querschnitt verspringt die Trennfläche derart, dass das eine äußere Teilelement 12 in Richtung des anderen Teilelementes 14 ragende Vorsprünge 26 aufweist, die in entsprechende Ausnehmungen 28 an dem anderen äußeren Teilelement 14 eingreifen. Diese Vorsprünge 26 beziehungsweise Ausnehmungen 28 sind so angeordnet, dass sich die beiden Teilelemente 12 und 14 weder im kompakten Zustand des Spreizelementes 10 (siehe Fig. 2) noch im expandierten Zustand (siehe Fig. 3) des Spreizelementes 10 in Längsrichtung oder in seitlicher Richtung relativ zueinander verschieben können.

Jeweils entlang der Längsrichtung der beiden äußeren Teilelemente 12 und 14 verlaufende Vertiefungen 30 beziehungsweise 32 definieren den Freiraum 18 zwischen den beiden äußeren Teilelementen 12 und 14, in den das Spreizelement 16 eingesetzt werden kann. Wie den Figuren zu entnehmen ist, besitzt der Freiraum 18 im Wesentlichen über seine gesamte Länge einen einheitlichen rechteckigen Querschnitt, der an den Ecken etwas abgerundet ist. Im Bereich des distalen Endes des Befestigungselementes 10 verjüngt sich der Freiraum 18 etwas.

Das Spreizelement 16 besitzt einen Querschnitt, der dem Querschnitt des Freiraums 18 im Wesentlichen entspricht. Jedoch ist die Ausdehnung des Spreizelementes 16 in der quer zur Trennfläche 24 verlaufenden Richtung etwas größer als die entsprechende Ausdehnung des Freiraums 18, damit das Spreizelement 16 das Befestigungselement 10 auch tatsächlich aufspreizt, wenn es in den Freiraum 18 eingeschoben wird. Damit sich das Spreizelement 16 ohne weiteres in den Freiraum 18 einschieben lässt, auch wenn sich das Befestigungselement 10 zunächst in seinem kompakten Zustand befindet, nimmt die entsprechende Ausdehnung des Spreizelementes 16 im Bereich des distalen Endes kontinuierlich bis auf ein Maß ab, das es erlaubt, das Spreizelement 16 mit seinem distalen Ende voran in den Freiraum 18 des Befestigungselementes in dessen kompakten Zustand einzuführen.

Die beiden äußeren Teilelemente 12 und 14 sowie das Spreizelement 16 bestehen jeweils aus einem bioresorbierbaren Thermoplast, vorzugsweise einem Polylactid, welches die Eigenschaft besitzt, dass es nach Implantation vom menschlichen Körper allmählich abgebaut wird, so dass nach Verheilung eines Bruches, der Mittels des Befestigungselementes 10 fixiert wurde, kein Implantat mehr aus dem menschlichen Körper entfernt werden muss. Der Werkstoff der beiden äußeren Teile 12 und 14 sowie des Spreizelementes 16 hat darüber hinaus die Eigenschaft, mit einem heißen Draht geschnitten werden zu können, so dass das Befestigungselement 10 in Bezug auf eine Bohrung in einem Knochen eine Überlänge besitzen und nach Implantation und Einführen des Spreizelementes 16 derart beschnitten werden kann. Ein außen über den Knochen überstehender Teil des Befestigungselementes 10 kann somit leicht abgeschnitten werden. Das Abschneiden erfolgt vorzugsweise mit einem Gerät, das einen elektrisch beheizten Schneiddraht aufweist, mit dem sich der thermoplastische Werkstoff des Befestigungselementes 10 leicht schneiden lässt. Dabei kommt es vorteilhafter Weise zu einer Verschweißung der beiden äußeren Teilelemente 12 und 14 mit dem Spreizelement 16 im Bereich der Schnittstelle.

Der Werkstoff der beiden äußeren Teilelemente 12 und 14 und gegebenenfalls auch des Spreizelementes ist darüber hinaus vorzugsweise mit einer knochenwachstumsstimulierenden Substanz bestückt, die das Befestigungselement 10 nach seiner Implantation in einer Dosis abgibt, die geeignet ist, das Knochenwachstum in einer für den Heilungsprozess förderlichen Weise zu stimulieren.

Nicht dargestellte Varianten des erfindungsgemäßen Befestigungselementes weichen von den in Figuren 1 bis 3 dargestellten Befestigungselement 10 vor allem hinsichtlich der Länge ab, die größer sein kann, als die des dargestellten Befestigungselementes 10. Auch geringfügige Abweichungen des Durchmessers sind möglich. So kann dieser bis zu 1 mm geringer oder bis zu 2mm größer sein, als der Durchmesser des dargestellten Befestigungselementes. Insgesamt besitzt das Befestigungselement aus den Figuren 1 bis 3 jedoch eine Geometrie, die in vorteilhafter Weise weitgehend für die beabsichtigte Anwendung, beispielsweise die Behandlung von Oberschenkelhalsbrüchen, optimiert ist.

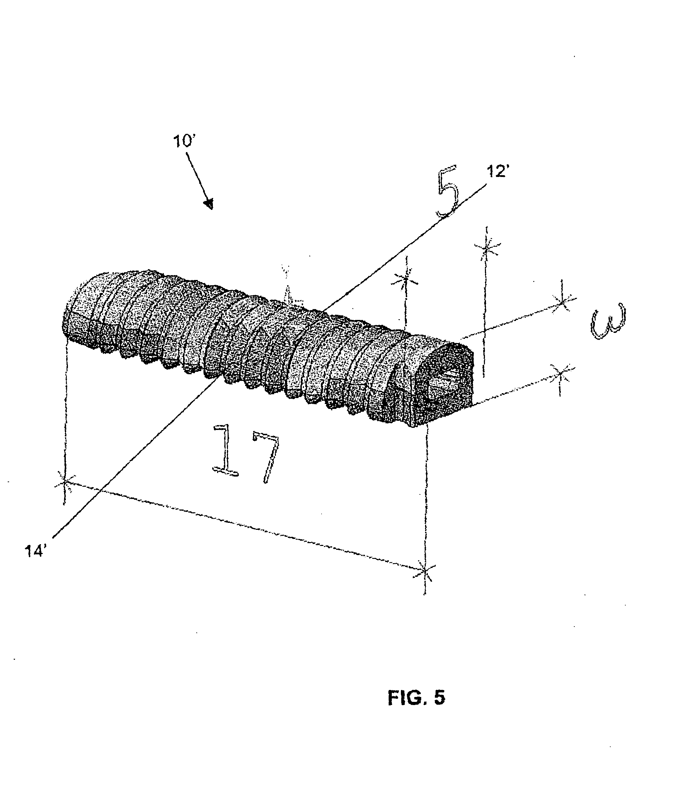

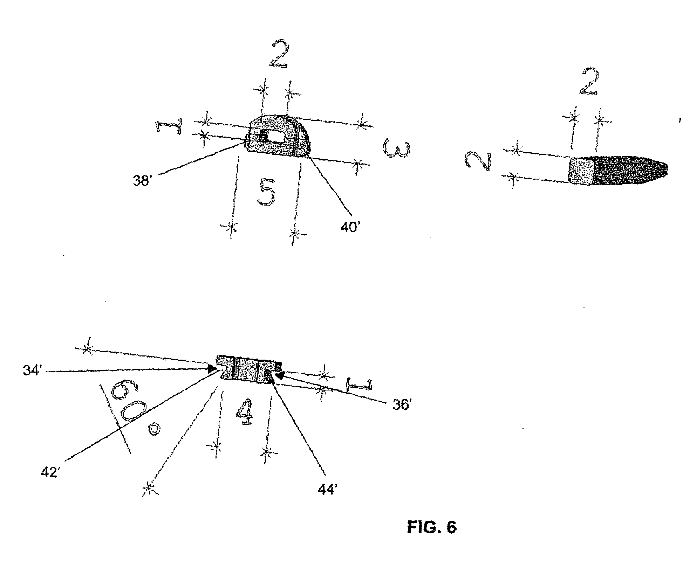

In Figuren 5 bis 7 ist ein alternatives Befestigungselement 10' abgebildet, welches im Unterschied zu den in Figuren 1 bis 4 abgebildeten Befestigungselement keinen kreisrunden Querschnitt besitzt, sondern auf der Seite eines der beiden Teilelemente 12' und 14' abgeflacht ist. Außerdem ist das in Figuren 5 bis 7 abgebildete Befestigungselement 10' kürzer, als das Befestigungselement gemäß Figuren 1 bis 4.

Das Befestigungselement 10' gemäß Figuren 5 bis 7 ist auch nicht für das miteinander Verbinden von Knochenfragmenten optimiert, sondern vielmehr für die Befestigung von Bändern an einem Knochen.

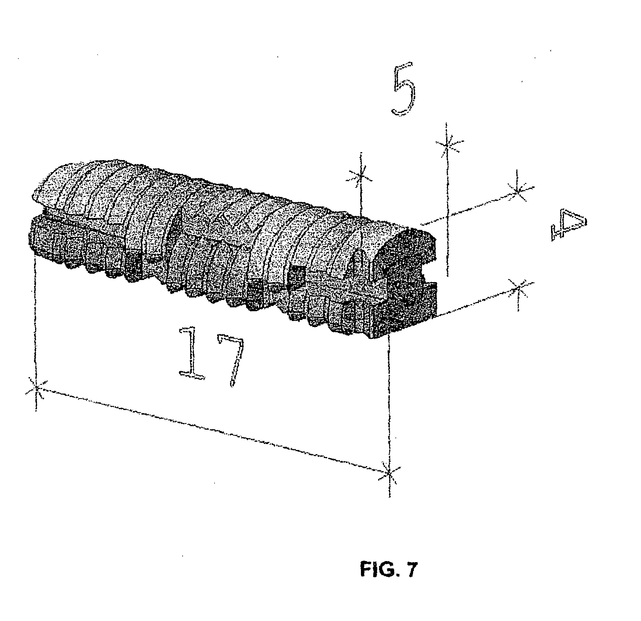

Wie den Figuren 5 und 7 zu entnehmen ist, besitzt das alternative Befestigungselement 10' an seinem proximalen Ende eine von zwei Kerben 34' und 36' gebildete Kopplungsstruktur, die der des in Figuren 1 bis 4 abgebildeten Befestigungselementes 10 entsprechen.

## Patentansprüche

1. Befestigungselement, insbesondere Dübel, für die Fixation von Knochenfragmenten, Bändern oder dergleichen, welches in Längsrichtung in wenigstens zwei äußere Teilelemente geteilt ist, die derart gestaltet und zueinander angeordnet sind, dass das Befestigungselement von einem kompakten Zustand in einen lateral oder radial expandierten Zustand zu überführen ist,
**dadurch gekennzeichnet, dass**
das Befestigungselement an seinem proximalen Ende zur Koppelung mit einem Instrument zum Halten und Einführen des Befestigungselementes schwalbenschwanzartig ausgebildet ist und zwei auf gegenüberliegenden Seiten des Befestigungselementes sich quer zu einer Trennfläche zwischen den Teilelementen erstreckende Kerben aufweist, die sich jeweils über beide Teilelemente hinweg erstrecken und die derart geformt sind, dass die Tiefe der jeweiligen Kerbe zum proximalen Ende des Befestigungselementes hin abnimmt und so die Breite des Befestigungselementes aus einer Blickrichtung entlang der beiden Kerben zum proximalen Ende des Befestigungselementes hin zunimmt, wobei die Breite des Befestigungselementes an dessen proximalem Ende geringer ist, als in einem Längsabschnitt, der sich in distaler Richtung an die Kerben anschließt.

2. Befestigungselement nach Anspruch 1, **dadurch gekennzeichnet, dass** die Kerben jeweils von einer distalen Grenzfläche, die senkrecht zur Längsachse des Befestigungselementes verläuft und von einer proximalen Grenzfläche, die in einem spitzen Winkel auf die Längsachse des Befestigungselementes zuläuft begrenzt sind.

3. Befestigungselement nach Anspruch 1 oder 2, **dadurch gekennzeichnet, dass** das Befestigungselement ein einem kompakten Zustand über den größten Teil seiner Länge eine zylindrische Grundform hat.

4. Befestigungselement nach Anspruch 3, **dadurch gekennzeichnet, dass** die Breite des Befestigungselementes an seinem proximalen Ende aufgrund der beiden Kerben um ein Maß geringer ist, als der Durchmesser der zylindrischen Grundform, das etwas mehr als der Tiefe einer Kerbe in radialer Richtung entspricht.

5. Befestigungselement nach Anspruch 3 oder 4, **dadurch gekennzeichnet, dass** die Teilelemente dem Befestigungselement in seinem kompakten Zustand eine zylindrische Grundform mit einem Durchmesser zwischen 3 und 7 mm und einer Länge von über 60 mm verleihen, und sich in Längsrichtung des Befestigungselements in seinem kompakten Zustand Längsabschnitte mit runden Querschnitt und solche mit sternförmigen Querschnitt, der jeweils von prismenförmigen Längsrippen auf der Außenseite dieser Längsabschnitte gebildet ist, abwechseln, wobei der Durchmesser der Längsabschnitte mit rundem Querschnitt geringer ist, als der Durchmesser der Längsabschnitte mit sternförmigem Querschnitt.

6. Befestigungselement nach Anspruch 5, **dadurch gekennzeichnet, dass** die Längsabschnitte mit sternförmigem Querschnitt eine Länge zwischen 1 und 3 mm haben.

7. Befestigungselement nach Anspruch 5 oder 6, **dadurch gekennzeichnet, dass** die Längsabschnitte mit rundem Querschnitt eine Länge zwischen 0,5 und 1,5 mm haben.

8. Befestigungselement nach einem der Ansprüche 5 bis 7, **dadurch gekennzeichnet, dass** die Längsabschnitte mit rundem Querschnitt Zylinderform haben.

9. Befestigungselement nach einem der Ansprüche 5 bis 8, **dadurch gekennzeichnet, dass** der Freiraum und das Spreizelement jeweils einen rechteckigen Querschnitt besitzen.

10. Befestigungselement nach Anspruch 9, **dadurch gekennzeichnet, dass** das Befestigungselement in Längsrichtung entlang einer Trennfläche zweigeteilt und somit zwei Teilelemente aufweist, die den Freiraum einschließen, wobei das Querschnittsmaß des Freiraums in einer parallel zum größten Teil der Trennfläche verlaufenden Richtung dem Querschnittsmaß des Spreizelementes entspricht und das Spreizelement in einer quer zum größten Teil der Trennfläche verlaufenden Richtung ein Querschnittsmaß aufweist, dass über den größten Teil der Länge des Spreizelementes gleich bleibend und größer als das Querschnittsmaß des Freiraums in der entsprechenden Richtung ist.

11. Befestigungselement nach Anspruch 10, **dadurch gekennzeichnet, dass** das Spreizelement an seinem distalen Ende keilförmig angespitzt ist und sich in distale Richtung bis auf eine Querschnittsmaß verjüngt, dass geringer ist als das Querschnittsmaß des Freiraum im kompakten Zustand des Befestigungselements.

12. Befestigungselement nach einem der Ansprüche 1 bis 11, **dadurch gekennzeichnet, dass** das Befestigungselement aus bioresorbierbarem Kunststoff besteht.

13. Befestigungselement nach Anspruch 12, **dadurch gekennzeichnet, dass** der bioresorbierbare Kunststoff ein Polylactid ist oder enthält.

14. Befestigungselement nach Anspruch 12 oder 13, **dadurch gekennzeichnet, dass** der bioresorbierbare Kunststoff ein Thermoplast ist, dass mittels eines heißen Drahtes zu schneiden ist.

15. Befestigungselement nach einem der Ansprüche 1 bis 14, **dadurch gekennzeichnet, dass** das Befestigungselement nach Implantation Osteosynthese stimulierende Substanzen abgibt.

## Claims

1. Fixing element, in particular a plug, for fixing bone fragments, ligaments or the like, which is divided in longitudinal direction into at least two outer sub-elements, which are designed and arranged relative to one another such that the fixing element can be converted from a compact state into a laterally or radially expanded state,
**characterised in that**
the fixing element has a swallow-tail form at its proximal end for coupling with an instrument for holding and introducing the fixing element and has two notches on opposite sides of the fixing element that extend at right angles to a separating surface between the sub-elements, which notches extend over both sub-elements and which are shaped in such a way that the depth of the respective notch reduces towards the proximal end of the fixing element, thus increasing the width of the fixing element, from one direction of view, along the two notches towards the proximal end of the fixing element, wherein the width of the fixing element at its proximal end is smaller than the width in a longitudinal section adjoining the notches in distal direction.

2. Fixing element according to claim 1, **characterised in that** the notches are delimited respectively by a distal boundary surface, which is perpendicular to the longitudinal axis of the fixing element and by a proximal boundary surface, which runs at an acute angle towards the longitudinal axis of the fixing element.

3. Fixing element according to claim 1 or 2, **characterised in that** the fixing element in a compact state has a cylindrical basic shape over the greatest part of its length.

4. Fixing element according to claim 3, **characterised in that** the width of the fixing element at its proximal end due to the two notches is smaller by an amount than the diameter of the cylindrical basic shape, which in radial direction is slightly more than the depth of a notch.

5. Fixing element according to claim 3 or 4, **characterised in that** the sub-elements give the fixing element in its compact state a cylindrical basic shape with a diameter of between 3 and 7 mm and a length of more than 60 mm, and in the longitudinal direction of the fixing element in its compact state longitudinal sections with a round cross section alternate with those with a star-shaped cross section, which is formed respectively by prism-like longitudinal ribs on the outside of said longitudinal sections, whereby the diameter of the longitudinal sections with a round cross section is smaller than the diameter of the longitudinal sections with a star-shaped cross section.

6. Fixing element according to claim 5, **characterised in that** the longitudinal sections with a star-shaped cross section have a length of between 1 and 3 mm.

7. Fixing element according to claim 5 or 6, **characterised in that** the longitudinal sections with a round cross section have a length of between 0.5 and 1.5 mm.

8. Fixing element according to one of claims 5 to 7, **characterised in that** the longitudinal sections with a round cross section have a cylindrical shape.

9. Fixing element according to one of claims 5 to 8, **characterised in that** the free space and the spreading element each have a rectangular cross section.

10. Fixing element according to claim 9, **characterised in that** the fixing element is divided into two in longitudinal direction along a separating surface and thus comprises two sub-elements which enclose the free space, wherein the cross sectional dimension of the free space in a direction running parallel to the greatest part of the separating surface corresponds to the cross sectional dimension of the spreading element, and the spreading element has a cross sectional dimension in a direction running at right angles to the greatest part of the separating surface, **in that** over the greatest part of the length of the spreading element it is consistent and greater than the cross sectional dimension of the free space in the corresponding direction.

11. Fixing element according to claim 10, **characterised in that** the spreading element is pointed at its distal end in a wedge shape and tapers in distal direction up to a cross sectional dimension which is smaller than the cross sectional dimension of the free space in the compact state of the fixing element.

12. Fixing element according to one of claims 1 to 11, **characterised in that** the fixing element is made of bioresorbable plastic.

13. Fixing element according to claim 12, **characterised in that** the bioresorbable plastic is a polylactide or contains the latter.

14. Fixing element according to claim 12 or 13, **characterised in that** the bioresorbable plastic is a thermoplastic which has to be cut by means of a hot wire.

15. Fixing element according to one of claims 1 to 14, **characterised in that** the fixing element after implantation emits osteosynthesis stimulating substances.

## Revendications

1. Elément de fixation, en particulier cheville, pour la fixation de fragments osseux, de plaques ou similaires, divisé en au moins deux éléments partiels externes dans le sens de la longueur, lesquels sont façonnés et disposés l'un par rapport à l'autre de telle sorte que l'élément de fixation puisse passer d'un état compact dans un état latéralement ou radialement expansé,
**caractérisé en ce que**
l'élément de fixation est formé en queue d'aronde à son extrémité proximale pour le couplage avec un instrument pour tenir et introduire l'élément de fixation, et présente deux rainures s'étendant en travers d'une surface de séparation entre les éléments partiels sur des côtés opposés de l'élément de fixation, rainures qui s'étendent respectivement sur les deux éléments partiels et sont formées de telle sorte que la profondeur de la rainure respective diminue en allant vers l'extrémité proximale de l'élément de fixation et donc que, partant d'une direction du regard le long des deux rainures, la largeur de l'élément de fixation augmente en allant vers l'extrémité proximale de l'élément de fixation, la largeur de l'élément de fixation étant plus faible à son extrémité proximale que dans un tronçon longitudinal qui se raccorde dans le sens distal aux rainures.

2. Elément de fixation selon la revendication 1, **caractérisé en ce que** les rainures sont respectivement limitées par une surface limite distale passant à la verticale de l'axe longitudinal de l'élément de fixation et par une surface limite proximale courant en angle aigu vers l'axe longitudinal de l'élément de fixation.

3. Elément de fixation selon la revendication 1 ou 2, **caractérisé en ce que** l'élément de fixation a, dans un état compact, une forme de base cylindrique sur la majeure partie de sa longueur.

4. Elément de fixation selon la revendication 3, **caractérisé en ce que** la largeur de l'élément de fixation est, en raison des deux rainures, plus faible à son extrémité proximale que le diamètre de la forme de base cylindrique dans une mesure équivalant à un peu plus que la profondeur d'une rainure dans le sens radial.

5. Elément de fixation selon la revendication 3 ou 4, **caractérisé en ce que** les éléments partiels confèrent à l'élément de fixation dans son état compact une forme de base cylindrique avec un diamètre compris entre 3 et 7 mm et une longueur de plus de 60 mm, et alternent dans le sens longitudinal de l'élément de fixation dans son état compact des tronçons longitudinaux à coupe transversale ronde et d'autres à coupe transversale en forme d'étoile, coupe respectivement formée par des nervures longitudinales en forme de prisme sur la face externe de ces tronçons longitudinaux, le diamètre des tronçons longitudinaux à coupe transversale ronde étant plus faible que le diamètre des tronçons longitudinaux à coupe transversale en forme d'étoile.

6. Elément de fixation selon la revendication 5, **caractérisé en ce que** les tronçons longitudinaux à coupe transversale en forme d'étoile ont une longueur comprise entre 1 et 3 mm.

7. Elément de fixation selon la revendication 5 ou 6, **caractérisé en ce que** les tronçons longitudinaux à coupe transversale ronde ont une longueur comprise entre 0,5 et 1,5 mm.

8. Elément de fixation selon l'une des revendications 5 à 7, **caractérisé en ce que** les tronçons longitudinaux à coupe transversale ronde ont la forme d'un cylindre.

9. Elément de fixation selon l'une des revendications 5 à 8, **caractérisé en ce que** l'espace libre et l'élément expansible ont respectivement une coupe transversale rectangulaire.

10. Elément de fixation selon la revendication 9, **caractérisé en ce que** l'élément de fixation est divisé en deux dans le sens de la longueur le long d'une surface de séparation et présente donc deux éléments partiels qui entourent l'espace libre, la dimension de coupe transversale de l'espace libre correspondant à la dimension de coupe transversale de l'élément expansible dans un sens parallèle à la majeure partie de la surface de séparation et l'élément expansible présentant une dimension de coupe transversale qui est constante sur la majeure partie de la longueur de la surface de séparation et supérieure à la dimension de coupe transversale de l'espace libre dans le sens correspondant.

11. Elément de fixation selon la revendication 10, **caractérisé en ce que** l'élément expansible est appointé en forme de coin à son extrémité distale et se rétrécit dans le sens distal jusqu'à une dimension de coupe transversale plus faible que la dimension de coupe transversale de l'espace libre dans l'état compact de l'élément de fixation.

12. Elément de fixation selon l'une des revendications 1 à 11, **caractérisé en ce que** l'élément de fixation est en plastique biorésorbable.

13. Elément de fixation selon la revendication 12, **caractérisé en ce que** le plastique biorésorbable est ou contient un polyactide.

14. Elément de fixation selon la revendication 12 ou 13, **caractérisé en ce que** le plastique biorésorbable est un thermoplastique à couper au moyen d'un fil chaud.

15. Elément de fixation selon l'une des revendications 1 à 14, **caractérisé en ce que** l'élément de fixation émet des substances stimulant l'ostéosynthèse après implantation.
